# EUROPÄISCHE PATENTSCHRIFT

(11) **EP 1 023 086 B1**
(45) Veröffentlichungstag und Bekanntmachung des Hinweises auf die Patenterteilung: **12.09.2007**
(21) Anmeldenummer: 99932862.8
(22) Anmeldetag: 10.07.1999
(51) Int. Cl.: A61L 2/00, A61L 15/40, A61L 15/46, A01N 65/00

(54) **DESINFIZIERENDER ANTIBAKTERIELLER HOLZPARTIKEL-SCHICHTSTOFF**
DISINFECTING ANTIBACTERIAL WOOD PARTICLE LAMINATE
MATIERE STRATIFIEE DESINFECTANTE ET ANTIBACTERIENNE, A BASE DE PARTICULES LIGNEUSES

(30) Priorität: 11.08.1998 DE 19836293
(43) Veröffentlichungstag der Anmeldung: 02.08.2000
(73) Patentinhaber: Firma Gustav Wilms, D-49152 Bad Essen (DE)
(72) Erfinder: WILMS, Heinrich, D-49328 Melle (DE)
(74) Vertreter: Busse & Busse Patentanwälte
(86) Internationale Anmeldenummer: PCT/EP1999/004850
(87) Internationale Veröffentlichungsnummer: WO 2000/009172

(56) Entgegenhaltungen:
- DE-A- 3 517 923
- DE-A- 3 518 562
- DE-A- 4 413 400
- FR-A- 2 682 710
- US-A- 3 238 056
- US-A- 4 748 048
- US-A- 4 883 663

## Beschreibung

Die Erfindung bezieht sich allgemein auf das Desinfizieren und Sterilisieren zur Schaffung hygienischer Bedinungen im täglichen Lebensmittelbereich und konkret auf eine Schicht zur Absorption und Abtötung von Bakterien, bestehend aus einer flachen Schicht aneinander haftender Holzpartikel in Gestalt von Holzschnitzeln, Holzspänen, Holzmehl und/oder Holzwolle.

Zum Desinfizieren oder Sterilisieren von Stoffen oder Gegenständen oder auch baulichen Einrichtungen sind Desinfektions- oder Sterilisationsverfahren und -vorrichtungen bekannt, die zur Erzielung der Keimfreiheit physikalische Erscheinung, z.B. Ultraschall, Wärme, insbesondere in Form von heißen Gasen oder auch Strahlung, z.B. Ultraviolett-Strahlung oder Mikrowellen, anwenden. Weitere Verwendung zum Desinfizieren oder Sterilisieren sind auch chemische Stoffe vorwiegend im flüssigen oder gasförmigen Zustand. Diese bekannten Verfahren erfordern überwiegend einen hohen apparativen Aufwand, der ihrer allgemeinen wirtschaftlichen Verwendung Grenzen setzt, wobei die chemischen Verfahren durch die verwendeten Substanzen in der Regel außerdem eine Umweltbelastung hervorrufen.

Aus der DE 44 13 400 A ist ein Verfahren und eine Vorrichtung zum Desinfizieren von Luft, insbesondere in Lüftungs- oder Klimatisierungssystemen von Gebäuden bekannt, wobei der Lufteinströmungsweg vorbestimmter Länge durch eine Schüttung feiner Holzteilchen als Desinfektionsmaterial aufgezwungen wird. Die Schüttung feiner Holzteilchen kann in einem schlauchförmigen. Behältnis im Inneren einer luftbeströmten Rohrleitung untergebracht sein oder eine luftdurchströmte Bodenschüttung in einem Behälter nach Art eines Wirbelbettes bilden. Die Verwendung feiner Holzteilchen stellt dabei der Luftholzoberflächenbereich in großem Umfang zur Verfügung, was eine rasche Aufnahme von in der Luft enthaltenen Bakterien wie Salmonellen oder Ecoli gewährleisten soll mit dem Ergebnis einer keimabtötenden Wirkung. Diese Maßnahme ist jedoch auf die Reinigung von luftdurchströmten Systemen beschränkt und kann nicht in anderen Lebensbereichen des täglichen Bedarfs eingesetzt werden.

Aus der US-A-3,238,056 sind mikroporöse Materialien bekannt und ein Verfahren zur Herstellung derartiger mikroporöser Materialien mit mikrofeinen Poren, die für Filterprozesse Einsatz finden sollen. Für eine Verwendung in alltäglichen Lebensbereichen sind diese Materialien so ohne weiteres nicht verwendbar.

Die DE 35 17 923 beschreibt ein Verfahren zur Aufbereitung von Atemluft durch Holzschüttungen durchzublasen, wobei die Holzschüttungen mit Feuchtespendern zur Intensivierung des natürlichen Holzduftes versehen werden können. Dazu sollen natürliche Hölzer mit tragenden Elementen wie Rahmen- oder Filterstoffen so in Verbindung gebracht werden, daß die Hölzer aufgrund ihrer Porösität als staub- und gasförmige Filterflächen genutzt werden zur gleichzeitigen Atemluftreinigung und zur Bereitstellung einer natürlichen Duftnote. Diese Anwendung bleibt allerdings auf die Verbesserung von Atemluft beschränkt und dient auch nur als Filtermaterial. Für sonstige Bereiche des täglichen Lebensbedarfs sind diese nicht geeignet.

Der Erfindung liegt die Aufgabe zugrunde, zur Schaffung hygienischer Bedingungen solche Maßnahmen einsetzen zu können, die eine antibakterielle Wirkung bei hoher Wirtschaftlichkeit in ihrer Anwendung unter Vermeidung einer Umweltbelastung enthalten. Diese Aufgabe wird durch eine Schicht gemäß Patentanspruch 1 gelöst.

Versuche haben gezeigt, daß von Holzpartikeln, die in Form von Holzschnitzeln, - spänen, -mehl und/oder -wolle vorliegen, eine antibakterielle Wirkung ausgeht. Dabei wurde gefunden, daß die Bakterien, z.B. E. coli, durch physikalische Kräfte in den Holzporen festgehalten werden. Das poröse und hygroskope Holz entzieht den Bakterien die für ihre Lebensprozesse und Vermehrung notwendige Feuchtigkeit. Es erfolgt dabei ein Abtöten der Mikroorganismen durch Absenken der Wasseraktivität. Die Partikularstruktur der erfindungsgemäßen Schicht, die freie Oberflächenbereiche der Holzpartikel darbietet, ermöglicht eine Absorption der Bakterien in erheblichem Umfang.

Je nach der Konzentration der Mikroorganismen auf den Holzpartikeln konnte bei den Versuchen schon nach wenigen Stunden eine vollständige Abtötung der von den Holzpartikeln absorbierten Bakterien, die praktisch unablösbar mit dem Holz verbunden bleiben, nachgewiesen werden. Den Holzinhaltsstoffen, z.B. den Polyphenolen, kommt dabei eine unterstützende Wirkung bei der Abtötung der Bakterien zu.

Da das Überleben der Bakterien wesentlich und unmittelbar an das Vorhandensein von frei verfügbarem Wasser in den Holzpartikeln gebunden ist, empfiehlt es sich, den Feuchtegehalt der Holzpartikel unterhalb des Fasersättigungsbereichs von etwa 7,5 % und nach Möglichkeit bei oder nahe 0 %, dem sog. darrtrockenen Zustand, zu halten. Dies schafft die Voraussetzung, daß hohe Bakterientiter von den Holzpartikeln absorbiert und in kurzer Zeit abgetötet werden können.

Dieser Vorgang läßt sich dadurch weiter günstig beeinflussen und beschleunigen, daß in weiterer Ausgestaltung der Erfindung für den Schichtstoff solche Holzpartikel verwendet werden, die mit einem Netzmittel (Tensid) und/oder einem Fettlöser imprägniert sind. Durch Netzmittel oder Tenside in Lösung wird die Oberflächen- bzw. Grenzflächenspannung des Wassers oder anderer Flüssigkeiten herabgesetzt, so daß diese in die Oberflächen der Holzpartikel eindringen und sie unter Verdrängung der Luft durchtränken und benetzen können. Die dadurch an die Holzpartikel gebundenen oberflächenaktiven Stoffe haben bei einem Bakterienbefall, üblicherweise durch Kontakt mit einer Bakterien in Suspension enthaltenden Flüssigkeit, ihrerseits eine bakterizide Wirkung durch Zerstörung der Zellmembran der Bakterien aufgrund der die Grenzflächen- bzw. Oberflächenspannung herabsetzenden Substanzen, Durch den ggf. zusätzlich vorhandenen Fettlöser wird diese Wirkung unterstützt. Als geeignete Netzmittel bzw. Tenside und ggf. Fettlöser kommen solche Stoffe in Betracht, wie sie z.B. in Wasch, Spül- und Reinigungsmitteln verwendet werden und dem Fachmann grundsätzlich bekannt sind, wobei biologisch gut abbaubare Stoffe, wie etwa Alkylpolyglukoside als Tensid, bevorzugt werden.

Bei den mit Partikeln von verschiedenen Holzarten (Nadelhölzer, Laubhölzer) durchgeführten Versuchen zeigte sich, daß auf allen getesteten Hölzern eine Keimzahlreduktion zu beobachten war. Vertreter der Familie der Pinaceae zeigten dabei jedoch die besten antibakteriellen Eigenschaften, wobei unter diesen der Titer der Testorganismen auf Kiefern- und Lärchenholzpartikeln mit Abstand am schnellsten abnahm. Ist daher vorgesehen, als Ausgangsmaterial für die Holzpartikel Hölzer aus der Familie der Pinaceae, insbesondere Kiefer und Lärche, zu verwenden.

Die erfindungsgemäß zu verwendende Schicht kann in ihrer einfachsten Ausführungsform aus einer einzigen Schicht der durch das Haftmittel untereinander verbundenen Holzpartikel bestehen. Es können aber auch eine oder mehrere weitere Einzelschichten von durch das Haftmittel untereinander verbundenen Holzpartikeln, ggf. mit unterschiedlicher Holzpartikelstruktur, auf einer solchen Holzpartikelgrundschicht abgelegt und mit dieser bzw. untereinander durch das Haftmittel verbunden sein.

In weiterer Ausgestaltung der Erfindung kann die Schicht eine Tragschicht aus einem flachen Trägermaterial umfassen.

Als flaches Trägermaterial für die erfindungsgemäß zu verwendende Schicht eignen sich Flachbahnen aus Kunststoffolie, insbesondere biologisch abbaubarer Kunststoffolie, Papier, Karton oder Pappe, die je nach Anwendungsfall auch zumindest einseitig eine thermoplastische Kunststoffschicht aufweisen oder umfassen können. Solche Schichtstoffe können nach Art von Tapeten, Gardinen oder Teppichen als Abdeck- oder Belagware in geschlossenen Räumen zur Aufrechterhaltung hygienischer Bedingungen verwendet werden. Hierfür kommen insbesondere Aufenthalts- und Behandlungsräume in Krankenhäusern und Sanatorien in Betracht. Aber auch die Stallungen von Viehzuchtbetrieben oder Viehtransporter sind ein bevorzugtes Einsatzgebiet für solche bahnförmigen antibakteriellen Schichtstoffe als Belag für Boden und Wände. Die Entsorgung nicht mehr verwendbaren, z.B. beschädigten, gebrauchten Schichtstoff-Bahnmaterials ist problemlos insbesondere durch Kompostierung möglich.

Sofern in weiterer Ausgestaltung der Erfindung die Schicht der Holzpartikel oberseitig von einer Abdeckschicht mit guter Gasdurchlässigkeit, z.B. einem Vlies oder einer perforierten Folie, abgedeckt ist, eröffnen sich zusätzliche Anwendungsmöglichkeiten eines solchen dreilagigen Schichtstoffes als Ausgangsmaterial zur Herstellung von Sanitärartikeln, z.B. Windeln und Papiertaschentüchern, oder auch von Verbandsmaterial. Als Vliesmaterial kommen die gebräuchlichen Vliesstoffe z.B. aus Kunststoffasern (Polypropylen, Polyester) oder, vorzugsweise, Viskose in Betracht.

Sofern die Abdeckschicht aus einem heißsiegelfähigen Material besteht, wie dies nach einem weiteren Gestaltungsmerkmal der Erfindung vorgesehen ist, kann die Abdeckschicht mit der Tragschicht in deren Ausgestaltung als Flachbahn bzw. Flachbahnzuschnitt mit einer thermoplastischen Kunststoffseite rundum unter Einschluß der Holzpartikelschicht heißversiegelt werden. Dies vermeidet bei einer Verwendung des Schichtstoffes z.B. als Windel oder Papiertaschentuch eine direkte Berührung der Haut mit den Holzpartikeln.

Ein Flachmaterial mit guter Gasdurchlässigkeit, wie Vlies, kann aber auch als flaches Trägermaterial für die Ablagerung bzw. Abstützung der Holzpartikelschicht verwendet werden.

Auch kann die Schicht als auswechselbare Einlage in einer Außenhülle aus Papier, Zellstoff oder dgl. Material verwendet werden. Dies ermöglicht im Bedarfsfall ein Auswechseln der Einlage bzw. der Außenhülle.

Hinsichtlich weiterer vorteilhafter Ausgestaltungen wird auf die Ansprüche 2 bis 11 verwiesen.

Eine solche Schicht kann dadurch hergestellt werden, daß auf einer ablösungsfähigen Unterlage flachbahnartig eine Schicht eines Gemisches von Holzpartikeln und pulverförmigem thermoplastischem Kunststoff ausgebreitet, wobei Klebstoffpulver durch Erwärmen in einen klebefähigen, die Holzpartikel bindenden Zustand überführt und nach dem Erhärten des Klebstoffes die Unterlage abgelöst wird. Die Unterlage kann hierbei nach Art einer Trennfolie ausgebildet sein, deren der Holzpartikelschicht zugewandte Oberseite eine solche, z.B. paraffinartige, Oberflächenbeschaffenheit aufweist, daß eine innige Verbindung der Unterlage mit der Holzpartikelschicht vermieden wird.

Auf diese Grundschicht des Gemisches von Holzpartikeln und thermoplastischem Kunststoff kann nach dessen Erhärten zumindest noch eine Schicht des pulverförmigen thermoplastischen Kunststoffes aufgebracht, dieser durch Erwärmen in einen klebefähigen Zustand überführt und darauf eine Schicht der Holzpartikel aufgebracht werden, um sich mit dem Klebstoff zu verbinden. Es kann so ein Holzpartikelschichtstoff aus mehreren Holzpartikeleinzelschichten gebildet werden.

Ein Verfahren zum Herstellen einer erfindungsgemäßen Schicht sieht daher vor, daß auf eine Flachbahn als Trägermaterial ein pulverförmiger thermoplastischer Klebstoff, aufgestreut, anschließend das Klebstoffpulver durch Erwärmen in einen klebefähigen Zustand überführt, sodann eine Schicht Holzpartikel auf den klebefähigen Klebstoff aufgestreut und abschließend die Bahn, nach einem Erhärten des Klebstoffes, auf eine Vorratsrolle gewickelt wird. Dabei kann auf die Holzpartikelschicht zumindest eine weitere Lage Klebstoffpulver, das durch Erwärmen klebefähig gemacht wird, und darauf eine Holzpartikelschicht, ggf. unter leichtem Andrücken an die Flachbahn, beispielsweise zwischen Walzenpaaren, aufgetragen werden.

In dieses Herstellungsverfahren kann auch das Aufbringen einer oberseitigen Abdeckschicht mit guter Gasdurchlässigkeit einbezogen sein, indem eine entsprechende Materialbahn von einer Vorratsrolle abgewickelt und auf die oder die zuletzt aufgetragene Holzpartikelschicht unter leichtem Andrücken, z.B. zwischen Walzenpaaren, aufgebracht und randseitig mit dieser verbunden wird.

Bei einem abgewandtelten Verfahren zum Herstellen einer erfindungsgemäßen Schicht ist vorgesehen, das von einer zumindest einseitig eine thermoplastische Kunststoffschicht aufweisenden Flachbahn als Trägermaterial ausgeht, wobei die thermoplastische Kunststoffschicht an ihrer Oberfläche durch Erwärmen klebefähig gemacht und darauf eine Holzpartikelschicht und ggf. eine äußere Abdeckschicht aufgebracht wird. Auch eine in dieser Weise mit der Holzpartikelschicht und ggf. der Abdeckschicht belegte Flachbahn wird nach dem Wiedererhärten des thermoplastischen Kunststoffs auf eine Vorratsrolle gewickelt, um in einer transportgünstigen Form an ihren Einsatzort verbracht werden zu können.

Die Verfahren zur Herstellung des antibakteriellen Schichtkörpers bewirken durch entsprechende Bemessung und Aufbringung des als Klebstoff bzw. Haftmittel Verwendung findenden thermoplastischen Klebstoffes (Hotmelt) in seinem klebefähigen Zustand nur eine vergleichsweise lockere gegenseitige Bindung der Holzpartikel untereinander und am Trägermaterial, damit die Partikularstruktur der Holzpartikel mit für eine Absorption von Bakterien freien Oberflächenbereichen der Holzpartikel erhalten bleibt. Mit dem gleichen Ziel wird das mit einer zumindest einseitig kunststoffbeschichteten Flachbahn als Trägermaterial arbeitende Verfahren eingesetzt, bei dem die durch Wärme erweichte Kunststoffschicht als Haftmittel wirkt und eine Schicht locker aufgestreuter Holzpartikel bindet.

Um seine antibakterielle Wirkung zu entfalten, benötigt der Schichtstoff nach der Erfindung keinerlei apparative Unterstützung, noch bedarf es einer Energiezufuhr, bei gleichzeitiger guter Umweltverträglichkeit aufgrund seiner Zusammensetzung aus Naturstoffen, die vorzugsweise auch die Basis für entsprechende Klebstoffe oder Haftmittel für die Holzpartikel bilden können

## Patentansprüche

1. Schicht zur Absorption und Abtötung von Bakterien, bestehend aus einer flachen Schicht aneinander haftender Holzpartikel in Gestalt von Holzschnitzeln, Holzspänen, Holzmehl und/oder Holzwolle, die mithilfe eines Klebstoffes oder dergleichen Haftmittel untereinander unter Erhalt ihrer freien Oberflächenbereiche darbietenden Partikularstruktur verbunden sind, wobei die Holzpartikel der Schicht von Hölzern aus der Familie der Pinaceae gebildet sind, und die Holzpartikel der Schicht mit einem Netzmittel (Tensid) und/oder mit einem Fettlöser imprägniert sind.

2. Schicht nach Anspruch 1, **dadurch gekennzeichnet, daß** die Schicht eine Tragschicht aus einem flachen Trägermaterial umfaßt.

3. Schicht nach einem der Ansprüche 1 oder 2, **dadurch gekennzeichnet, daß** die Holzpartikel von Hölzern der Kiefer und/oder der Lärche gebildet sind.

4. Schicht nach einem der Ansprüche 1 bis 3, **dadurch gekennzeichnet, daß** der Feuchtegehalt des Partikels unterhalb des Fasersättigungsbereichs liegt.

5. Schicht nach Anspruch 4, **dadurch gekennzeichnet, daß** der Feuchtegehalt der Holzpartikel bei 0 % oder ca. 0 % liegt.

6. Schicht nach einem der Ansprüche 1 bis 5, **dadurch gekennzeichnet, daß** das flache Trägermaterial von Flachbahnen aus biologisch abbaubarer Kunststoffolie, aus Papier, aus Karton oder aus Pappe gebildet ist.

7. Schicht nach Anspruch 6, **dadurch gekennzeichnet, daß** die Flachbahnen zumindest einseitig eine thermoplastische Kunststoffschicht aufweisen.

8. Schicht nach einem der Ansprüche 1 bis 7, **dadurch gekennzeichnet, daß** das Trägermaterial für die Holzpartikelschicht von einem gasdurchlässigen Flachmaterial gebildet ist.

9. Schicht nach einem der Ansprüche 1 bis 8, **dadurch gekennzeichnet, daß** die Holzpartikelschicht oberseitig von einer gasdurchlässigen Abdeckschicht abgedeckt ist.

10. Schicht nach Anspruch 9, **dadurch gekennzeichnet, daß** die Abdeckschicht aus Vlies besteht.

## Claims

1. Layer for the absorption and killing of bacteria, comprising a flat layer of wood particles, adhering to one another, in the form of wood chippings, wood shavings, sawdust and/or wood wool, which with the aid of a glue or similar adhesive are joined to one another whilst preserving their particular structure offering free surface areas, wherein the wood particles of the layer are formed from woods from the family of *Pinaceae,* and the wood particles of the layer are impregnated with a wetting agent (surfactant) and/or with a fat solvent.

2. Layer according to claim 1, **characterised in that** the layer comprises a carrier layer of a flat carrier material.

3. Layer according to one of the claims 1 or 2, **characterised in that** the wood particles are formed from woods from pine and/or larch.

4. Layer according to one of the claims 1 to 3, **characterised in that** the moisture content of the particle lies below the fibre saturation range.

5. Layer according to claim 4, **characterised in that** the moisture content of the wood particles lies at 0% or approximately 0%.

6. Layer according to one of the claims 1 to 5, **characterised in that** the flat carrier material is formed by flat lengths of biodegradable synthetic film, paper, carton or cardboard.

7. Layer according to claim 6, **characterised in that** the flat lengths have a thermoplastic synthetic layer at least on one side.

8. Layer according to one of the claims 1 to 7, **characterised in that** the carrier material for the wood particle layer is formed by a gas-permeable flat material.

9. Layer according to one of the claims 1 to 8, **characterised in that** the wood particle layer is covered on the top with a gas-permeable covering layer.

10. Layer according to claim 9, **characterised in that** the covering layer comprises non-woven material.

## Revendications

1. Couche d'absorption et de destruction de bactéries, consistant en une couche plane de particules ligneuses adhérant les unes aux autres, sous la forme de copeaux de bois, d'éclisses de bois, de fibres de bois et / ou de laine de bois, et qui sont liées les unes aux autres au moyen d'un adhésif ou d'un agent de solidarisation similaire en conservant leur structure particulaire présentant des plages de surface libres, les particules ligneuses de la couche étant formées de bois de la famille des pinacées et les particules ligneuses de la couche étant imprégnées d'un agent mouillant (tensioactif) et / ou d'un solvant de graisse.

2. Couche suivant la revendication 1, **caractérisée en ce que** la couche comprend une couche portante d'un matériau de support plan.

3. Couche suivant l'une quelconque des revendications 1 ou 2, **caractérisée en ce que** les particules ligneuses sont formées de bois de pin et / ou de mélèze.

4. Couche suivant l'une quelconque des revendications 1 à 3, **caractérisée en ce que** la teneur en humidité des particules se situe au-dessous de la plage de saturation des fibres.

5. Couche suivant la revendication 4, **caractérisée en ce que** la teneur en humidité des particules ligneuses est de 0% ou d'environ 0%.

6. Couche suivant l'une quelconque des revendications 1 à 5, **caractérisée en ce que** le matériau de support plan est formé de bandes planes en feuille de matière synthétique biodégradable, en papier, en carton ou en carton-pâte.

7. Couche suivant la revendication 6, **caractérisée en ce que** les bandes planes présentent sur au moins une face une couche de matière synthétique thermoplastique.

8. Couche suivant l'une quelconque des revendications 1 à 7, **caractérisée en ce que** le matériau de support de la couche de particules ligneuses est formé d'un matériau plat perméable aux gaz.

9. Couche suivant l'une quelconque des revendications 1 à 8, **caractérisée en ce que** la couche de particules ligneuses est recouverte, sur sa face supérieure, d'une couche de couverture perméable aux gaz.

10. Couche suivant la revendication 9, **caractérisée en ce que** la couche de couverture est en non tissé.
